# EUROPÄISCHE PATENTANMELDUNG

(11) **EP 1 080 716 A1**
(43) Veröffentlichungstag der Anmeldung: **07.03.2001**
(21) Anmeldenummer: 00117050.5
(22) Anmeldetag: 09.08.2000
(51) Int. Cl.: A61K 7/48

(54) **Verwendung von anellierten Benzolderivaten als Antioxidans oder Radikalfänger in kosmetischen Zubereitungen**

(30) Priorität: 01.09.1999 DE 19941595
(71) Anmelder: BASF AKTIENGESELLSCHAFT, 67056 Ludwigshafen (DE)
(72) Erfinder: Streicher, Harald, Dr., 68181 Mannheim (DE)

(57) **Zusammenfassung**

Verwendung von anellierten Benzolderivaten der allgemeinen Formel I, 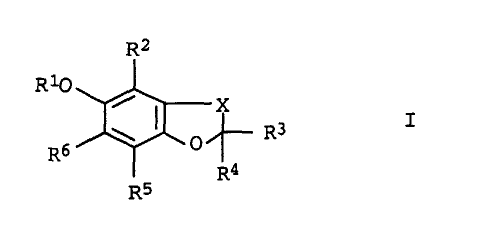 in der die Substituenten unabhängig voneinander folgende Bedeutung haben:
- R¹: Wasserstoff, C₁-C₁₂-Acyl;
- R²: Wasserstoff, C₁-C₁₂-Alkyl;
- R³ und R⁴: C₁-C₁₂-Alkyl;
- R⁵ und R⁶: Wasserstoff, C₁-C₁₂-Alkyl;
- X: -CH₂-, -(CH₂)₂-,
als Antioxidans und/oder Radikalfänger in kosmetischen oder dermatologischen Zubereitungen.

## Beschreibung

Die vorliegende Erfindung betrifft die Verwendung von anellierten Benzolderivaten als Antioxidans oder Radikalfänger in kosmetischen Zubereitungen. Ferner betrifft die Erfindung kosmetische und dermatologische Zubereitungen mit einem wirksamen Gehalt an anellierten Benzolderivaten. Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

In einer bevorzugten Ausführungsform betrifft die vorliegende Erfindung kosmetische und dermatologische Zubereitungen zur Prophylaxe und Behandlung kosmetischer oder dermatologischer Hautveränderungen wie z. B. der Hautalterung, insbesondere der durch oxidative Prozesse hervorgerufenen Hautalterung.

Weiterhin betrifft die vorliegende Erfindung Zubereitungen, enthaltend mindestens ein anelliertes Benzolderivat, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung betrifft auch Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UV-C-Bereich), von der Ozonschicht in der Erdatmosphäre absorbiert werden, verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich u.a. um Derivate des 3-Benzylidencamphers der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, dem sogenannten UV-A-Bereich, ist es wichtig, Filtersubstanzen zur Verfügung zu haben, da dessen Strahlen Reaktionen bei lichtempfindlicher Haut hervorrufen können. Es ist erwiesen, daß UV-A-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UV-B-Strahlung kann durch UV-A-Strahlung verstärkt werden.

Zum Schutz gegen die Strahlen des UV-A-Bereichs werden daher u.a. Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen können.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen auslösen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger zugesetzt werden.

Es ist bereits vorgeschlagen worden, Vitamin E - eine Substanz mit bekannter antioxidativer Wirkung - in Lichtschutzformulierungen einzusetzen, dennoch bleibt auch hier die erzielte Wirkung weit hinter der erhofften zurück.

Aufgabe der Erfindung war es daher, kosmetische und dermatologische Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z. B. A. Voelckel et al, Zentralblatt Haut- und Geschlechtskrankheiten (1989), 156, S. 2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Irjury" in "Oxidative Stress in Dermatology", S. 323 ff. (Marcel Decker Inc., New York, Basel, Hong Kong, Herausgeber: Jürgen Fuchs, Frankfurt und Lester Packer, Berkeley/Californien), werden oxidative Schäden der Haut und ihre näheren Ursachen aufgeführt.

Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar.

Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Antioxidantien sind Substanzen, welche Oxidationsprozesse verhindern bzw. welche die Autoxidation ungesättigte Verbindungen enthaltender Fette verhindern. Antioxidantien, welche auch auf dem Gebiete der Kosmetik und der Pharmazie Verwendung finden, sind beispielsweise α-Tocopherol insbesondere in Form des α-Tocopherylacetats, Sesamöl, Callensäurederivate, Butylhyclroxyanisol und Butylhydroxytoluol.

Auch aus dem Grunde, solchen Reaktionen vorzubeugen, können kosmetischen Formulierungen zusätzlich Antioxidantien und/oder Radikalfänger zugesetzt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E in Lichtschutzformulierungen einzusetzen.

Eine Aufgabe der vorliegenden Erfindung war, die Nachteile des Standes der Technik zu beseitigen. Insbesondere sollten Zubereitungen zur Verfügung gestellt werden, bei deren Verwendung die Schädigung der Haut und/oder des Haares durch oxidativen Einfluß zumindest gemindert, wenn nicht gänzlich verhindert werden können.

Eine weitere Aufgabe der vorliegenden Erfindung bestand darin, kosmetische Zubereitungen zur Verfügung zu stellen, welche vor oder nach Behandlung des Haars mit Haarfärbezubereitungen, selbst solcher mit einem Gehalt an starken Oxidationsmitteln wie z. B. Wasserstoffperoxid, deren schädigenden Oxidationswirkung entgegenwirken.

Diese Aufgaben wurden gelöst durch Verwendung von anellierten Benzolderivaten der allgemeinen Formel I, 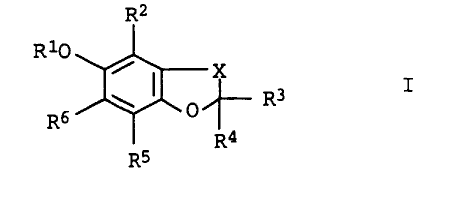 in der die Substituenten unabhängig voneinander folgende Bedeutung haben:
- R¹: Wasserstoff, C₁-C₁₂-Acyl;
- R²: Wasserstoff, C₁-C₁₂-Alkyl;
- R³ und R⁴: C₁-C₁₂-Alkyl;
- R⁵ und R⁶: Wasserstoff, C₁-C₁₂-Alkyl;
- X: -CH₂-, -(CH₂)₂-,
als Antioxidans und/oder Radikalfänger in kosmetischen oder dermatologischen Zubereitungen.

Als Acylreste für R¹ sind verzweigte oder unverzweigte, gesättigte oder ungesättigte, gegebenenfalls mehrfach ungesättigte C₁-C₁₂-Acylketten zu verstehen.

Beispiele hierfür sind Acylreste der Ameisen-, Essig-, Propion-, n-Butter-, iso-Butter-, Sorbin-, n-Valerian-, iso-Valerian-, Capron-, Capryl-, Caprin-, Undecan-, Laurinsäure.

Als bevorzugte Reste für R¹ seien Wasserstoff sowie Acetyl, besonders bevorzugt Wasserstoff genannt.

Als Alkylreste für R² bis R⁶ seien verzweigte oder unverzweigte C₁-C₁₂-Alkylketten, beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, n-Nonyl, n-Decyl, n-Undecyl und n-Dodecyl genannt.

Als bevorzugte Alkylreste für R² und R⁶ seien aus der o.g. Liste die verzweigten oder unverzweigten C₃-C₆-Alkylketten wie z.B. n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl genannt. Besonders bevorzugter Rest für R² und R⁶ ist der tert.-Butylrest.

Als bevorzugte Alkylreste für R³ und R⁴ seien aus der o.g. Liste die verzweigten oder unverzweigten C₃-C₈-Alkylketten wie z.B. n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, n-Heptyl, n-Octyl oder 2-Ethylhexyl genannt. Besonders bevorzugte Reste für R³ und R⁴ sind n-Pentyl, n-Hexyl und n-Octyl.

Ganz besonders bevorzugt ist die Verwendung von 2,3-Dihydro-5-hydroxy-2,2-dipentyl-4,6-di-tert.-butylbenzofuran der Formel Ia.

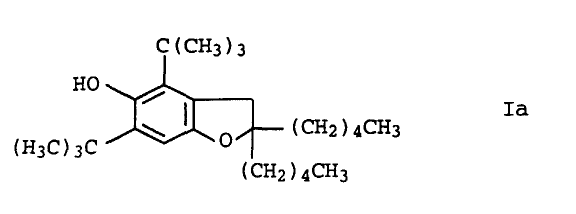

Insbesondere sollten Zukereitungen, solche Wirkstoffe der Formel I enthaltend, zur kosmetischen und dermatologischen Behandlung und/oder Prophylaxe erythematöser, entzündlicher, allergischer oder autoimmunreaktiver Erscheinungen, insbesondere Dermatosen, aber auch des Erscheinungsbildes des "Stingings" zur Verfügung gestellt werden.

WO 94/08930 beschreibt die Synthese sowie die antioxidativen Eigenschaften anellierter Benzolderivate und deren Verwendung als Mittel u.a. gegen Arteriosklerose. Noguchi et al. (Archieves of Biochemistry and Biophysics, Vol. 342, No. 2 (1997), 236-243 und Vol. 347, No. 1 (1997), 141-147) beschreibt die antioxidativen Eigenschaften von 2,3-Dihydro-5-hydroxy-2,2-dipentyl-4,6-di-tert. butylbenzofuran zum Schutz vor Lipid Peroxidation.

Es war indes überraschend und für den Fachmann nicht vorherzusehen, daß die Verwendung von anellierte Benzolderivate der Formel I als Antioxidans und/oder als Radikalfänger in kosmetischen oder dermatologischen Zubereitungen den Nachteilen des Standes der Technik abhelfen.

Kein Hinweis ist diesen Schriften zu entnehmen, welcher in die Richtung der vorliegenden Erfindung weisen könnte.

Es war für den Fachmann daher nicht vorauszusehen gewesen, daß die erfindungsgemäß verwendeten anellierten Benzolderivate der Formel I bzw. kosmetische oder dermatologische Zubereitungen, diese enthaltend
- besser gegen die Hautalterung wirken
- besser die Haut gegen Photoreaktionen schützen
- besser entzündlichen Hautreaktionen vorbeugen würde
als die Wirkstoffe, Wirkstoffkombinationen und Zubereitungen des Standes der Technik. Ferner war nicht vorauszusehen gewesen, daß die anellierten Benzolderivate der Formel I in kosmetischen oder dermatologischen Zubereitungen höhere Stabilität aufweisen als vergleichbare Wirkstoffe, beispielsweise als Vitamin C.

Erfindungsgemäß sind daher die Verwendung von anellierten Benzolderivaten der Formel I als Antioxidans sowie seine Verwendung zur Bekämpfung und/oder Prophylaxe der durch oxidative Beanspruchung hervorgerufenen Hautalterung und entzündlicher Reaktionen.

Die erfindungsgemäßen kosmetischen oder dermatologischen Formulierungen können wie üblich zusammengesetzt sein und zur Behandlung, der Pflege und der Reinigung der Haut und/oder der Haare und als Schminkprodukt in der dekorativen Kosmetik dienen. Sie enthalten in der Regel 0,01 bis 15 Gew.-%, bevorzugt 0,05 bis 5 Gew.-%, insbesondere 0,1 bis 2,0 Gew.-%, bezogen auf das Gesamtgewicht des Mittels, an mindestens einem anellierten Benzolderivat der Formel I.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch vorteilhaft, die anellierten Benzolderivate der Formel I in verkapselter Form darzureichen, z.B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, anellierte Benzolderivate der Formel I in wäßrige Systeme bzw. Tensidzubereitungen zur Reinigung der Haut und der Haare einzufügen.

Als vorteilhafte Verkörperung der vorliegenden Erfindung wird daher auch die Verwendung von mindestens einem anellierten Benzolderivat der Formel I zum Schutze der Haut und/oder der Haare vor oxidativer Beanspruchung angesehen, insbesondere die Verwendung mindestens eines anellierten Benzolderivates der Formel I in Shampoos und Waschformulierungen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können die anellierten Benzolderivate der Formel I erfindungsgemäß auch mit anderen Antioxidantien und/oder Radikalfängern kombiniert werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄), Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise (Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäβ zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol Ethylenglykolmonoethyl- oder -monobutylether, Propylenglykolmonomethyl, -monoethyl- oder monobutylether, Diethylenglykolmonomethyl- oder monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel. welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen, vorzugsweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UV-A- und UV-B-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Zubereitungen UV-B-Filtersubstanzen, können diese öllöslich oder wasserlöslich sein. Erfindungsgemäß vorteilhafte öllösliche UV-B-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)-benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, -2,4,6-Trianilino-(p-carbo-2'-ethyl-1'-hexyloxy)-1,3,5-triazin.

Vorteilhafte wasserlösliche UV-B-Filter sind z. B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-Di(2-oxo-10-Sulfo-3-bornylidenmethyl)-Benzol und dessen Salze (die entsprechenden 10-Sulfatoverbindungen, beispielsweise das entsprechende Natrium-, Kalium- oder Triethanolammonium-Salz), auch als Benzol-1,4-di(2-oxo-3-bornylidenmethyl)-10-Sulfonsäure bezeichnet.

Die Liste der genannten UV-B-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination mindestens eines anellierten Benzolderivates der Formel I mit mindestens einem UV-B-Filter als Antioxidans bzw. die Verwendung einer Kombination mindestens eines anellierten Benzolderivates der Formel I mit mindestens einem UV-B-Filter als Antioxidans in einer kosmetischen oder dermatologischen Zubereitung.

Es kann auch von Vorteil sein, mindestens ein anelliertes Benzolderivat der Formel I mit UV-A-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-iso-propylphenyl)propan-1,3-dion. Auch diese Kombinationen bzw. Zubereitungen, die diese Kombinationen enthalten, sind Gegenstand der Erfindung. Es können die für die UV-B-Kombination verwendeten Mengen eingesetzt werden.

Gegenstand der Erfindung ist auch die Verwendung einer Kombination mindestens eines anellierten Benzolderivates der Formel I mit mindestens einem UV-A-Filter als Antioxidans bzw. die Verwendung einer Kombination mindestens eines anellierten Benzolderivates der Formel I mit mindestens einem UV-A-Filter als Antioxidans in einer kosmetischen oder dermatologischen Zubereitung.

Gegenstand der Erfindung ist auch die Verwendung einer Kombination aus mindestens einem anellierten Benzolderivat der Formel I mit mindestens einem UV-A-Filter und mindestens einem UV-B-Filter als Antioxidans bzw. die Verwendung einer Kombination aus mindestens einem anellierten Benzolderivat der Formel I mit mindestens einem UV-A-Filter und mindestens einem UV-B-Filter als Antioxidans in einer kosmetischen oder dermatologischen Zubereitung.

Kosmetische und dermatologische Zubereitungen mit einem wirksamen Gehalt an mindestens einem anellierten Benzolderivat der Formel I können auch anorganische Pigmente enthalten, die üblicherweise in der Kosmetik zum Schutze der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UV-A-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektrolyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate. Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, anellierte Benzolderivate der Formel I im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie mindestens ein anelliertes Benzolderivat der Formel I in wirksamer Konzentration. Die Menge der verwendeten Polymeren liegt z.B. zwischen 0,1 und 10 Gew.-%, bevorzugt zwischen 0,1 und 3 Gew.-%.

Kosmetische Zubereitungen zur Behandlung und Pflege der Haare, die mindestens ein anelliertes Benzolderivat der Formel I enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an mindestens einem anellierten Benzolderivat der Formel I und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge an anellierten Benzolderivaten der Formel I in einem für die Haare bestimmten Mittel 0,01 bis 15 Gew.-%, bevorzugt 0,05 bis 5 Gew.-%, insbesondere 0,1 bis 2 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine Fettsäurealkanolamide
- Polyglycolether-Derivate.

Kosmetische Zubereitungen, die kosmetische Reinigungszubereitungen für die Haut darstellen, können in flüssiger oder fester Form vorliegen. Sie enthalten neben mindestens einem anellierten Benzolderivat der Formel I vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an mindestens einem anellierten Benzolderivat der Formel I vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die vorliegende Erfindung umfaßt auch ein kosmetisches Verfahren zum Schutze der Haut und der Haare vor oxidativen bzw. photooxidativen Prozessen, das dadurch gekennzeichnet ist, daß man ein kosmetisches Mittel, welches eine wirksame Konzentration an mindestens einem anellierten Benzolderivat der Formel I enthält, in ausreichender Menge auf die Haut oder Haare aufbringt.

Ebenso umfaßt die vorliegende Erfindung auch ein Verfahren zum Schutze kosmetischer oder dermatologischer Zubereitungen gegen Oxidation oder Photooxidation, wobei diese Zubereitungen z.B. Zubereitungen zur Behandlung und Pflege der Haare darstellen, insbesondere Haarfärbemittel, Haarlacke, Shampoonierungsmittel, Farbshampoonierungsmittel, ferner Schminkprodukte wie z.B. Nagellacke, Lippenstifte, Teintgrundlagen, Wasch- und Duschzubereitungen, Cremes zur Behandlung oder Pflege der Haut oder um sämtliche anderen kosmetischen Zubereitungen handelt, deren Bestandteile Stabilitätsprobleme aufgrund von Oxidation bzw. Photooxidation bei der Lagerung mit sich bringen können, dadurch gekennzeichnet, daß die kosmetischen Zubereitungen einen wirksamen Gehalt an mindestens einem anellierten Benzolderivat der Formel I aufweisen.

Vorzugsweise beträgt die Menge an anellierten Benzolderivaten der Formel I in diesen Zubereitungen 0,01 bis 15 Gew.-%, bevorzugt 0,05 bis 5 Gew.-%, insbesondere 0,1 bis 2,0 Gew.-%, bezogen auf das Gesamtgewicht der Zubereitungen.

Gegenstand der Erfindung ist auch das Verfahren zur Herstellung der erfindungsgemäßen kosmetischen Mittel, das dadurch gekennzeichnet ist, daß man in an sich bekannter Weise erfindungsgemäßen Wirkstoffkombinationen in kosmetische und dermatologische Formulierungen einarbeitet.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1

### O/W Lotion

| | Gew.-% |
|---|---|
| Paraffinöl (DAB 9) | 8,00 |
| Isopropylpalmitat | 3,00 |
| Petrolatum | 4,00 |
| Cetylstearyalkohol | 2,00 |
| PEG-40 Rizinusöl | 0,50 |
| Natriumcetylstearylsulfat | 0,5 |
| Natrium Carbomer | 0,40 |
| Verbindung Ia | 0,50 |
| Glycerin | 3,00 |
| α-Tocopherol | 0,20 |
| Octylmethoxycinnamat | 5,00 |
| Butylmethoxydibenzoylmethan | 1,00 |
| Konservierungsmittel, Farbstoffe, Parfüm | q.s. |
| Wasser | ad 100,00 |

### Beispiel 2

### Lippenpflegestift

| | Gew.-% |
|---|---|
| Hydriertes Rizinusöl | 4,00 |
| Ceresin | 8,00 |
| Bienenwachs | 4,00 |
| Carnaubawachs | 2,00 |
| Petrolatum | 40,00 |
| Verbindung Ia | 0,50 |
| β-Carotin | 0,10 |
| Konservierungsmittel, Farbstoffe, Parfüm | q.s. |
| Paraffinöl | ad 100,00 |

### Beispiel 3

### Gel

| | Gew.-% |
|---|---|
| Carbopol 934 P | 2,00 |
| Triethanolamin | 3,00 |
| Verbindung Ia | 0,50 |
| α-Tocopherylacetat | 0,20 |
| Polyoxyethylensorbitanfettsäureester (Tween 20) | 0,50 |
| Glycerin | 2,00 |
| Natrium PCA | 0,50 |
| Hydrolysiertes Kollagen | 2,00 |
| Konservierungsmittel, Farbstoffe, Parfüm | q.s. |
| Wasser | ad 100,00 |

### Beispiel 4

### Sonnenschutzemulsion

| | Gew.-% |
|---|---|
| Cyclomethicon | 2,00 |
| Cetyldimethicon Copolyol | 0,20 |
| PEG 22-Dodecyl Copolymer | 3,00 |
| Paraffinöl (DAB 9) | 2,00 |
| Caprylsäure-/Caprinsäure Triglycerid | 5,80 |
| Octylmethoxycinnamat | 5,80 |
| Butyl-methoxy-dibenzoylmethan | 4,00 |
| Verbindung Ia | 0,50 |
| α-Tocopherylacetat | 0,50 |
| ZnSO₄ | 0,70 |
| Na₃HEDTA | 0,3 |
| Konservierungsmittel, Farbstoffe, Parfüm | q.s. |
| Wasser | ad 100,00 |

### Beispiel 5

Haarwasser

| | Gew.-% |
|---|---|
| Ethanol | 40,00 |
| Diisopropyladipat | 0,10 |
| PEG-40 hydriertes Rizinusöl | 0,20 |
| Verbindung Ia | 0,50 |
| α-Tocopherylacetat | 0,10 |
| Konservierungsmittel, Farbstoffe, Parfüm | q.s. |
| Wasser | ad 100,00 |

## Patentansprüche

1. Verwendung von anellierten Benzolderivaten der allgemeinen Formel I, 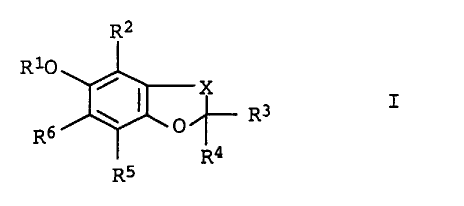 in der die Substituenten unabhängig voneinander folgende Bedeutung haben:
R¹ Wasserstoff, C₁-C₁₂-Acyl;
R² Wasserstoff, C₁-C₁₂-Alkyl;
R³ und R⁴ C₁-C₁₂-Alkyl;
R⁵ und R⁶ Wasserstoff, C₁-C₁₂-Alkyl;
X -CH₂-, -(CH₂)₂-,
als Antioxidans und/oder Radikalfänger in kosmetischen oder dermatologischen Zubereitungen.

2. Verwendung nach Anspruch 1, in der die Substituenten unabhängig voneinander folgende Bedeutung haben:
R¹ Wasserstoff;
R² C₃-C₆-Alkyl;
R³ und R⁴ C₃-C₈-Alkyl;
R⁵ Wasserstoff;
R⁶ C₃-C₆-Alkyl;
X -CH₂-.

3. Verwendung nach einem der Ansprüche 1 oder 2, dadurch gekennzeichnet, daß mindestens eine der Verbindungen der Formel I in einem wirksamen Gehalt in kosmetischen oder dermatologischen Zubereitungen vorliegt.

4. Verwendung nach einem der Ansprüche 1 bis 3, dadurch gekennzeichnet, daß mindestens eine der Verbindungen der Formel I in kosmetischen oder dermatologischen Zubereitungen in Konzentrationen von 0,01 bis 15 Gew.-% vorliegt.

5. Kosmetische oder dermatologische Zubereitungen zum Schutz der menschlichen Epidermis, dadurch gekennzeichnet, daß sie in einem kosmetisch und pharmazeutisch geeigneten Träger wirksame Mengen von Verbindungen der Formel I 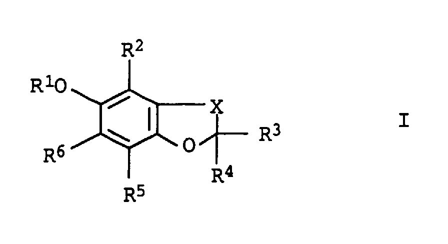 als Antioxidans oder Radikalfänger enthalten, in der die Variablen die Bedeutung definiert gemäß Anspruch 1 haben.

6. Kosmetische oder dermatologische Zubereitungen nach Anspruch 5, enthaltend als Antioxidans oder Radikalfänger Verbindungen der Formel I, in der die Variablen die Bedeutung definiert gemäß Anspruch 2 haben.

7. Kosmetische oder dermatologische Zubereitungen nach einem der Ansprüche 5 oder 6, enthaltend als Antioxidans oder Radikalfänger die Verbindungen der Formel Ia.
